(19) Europäisches Patentamt
European Patent Office
Office européen des brevets

(11) Veröffentlichungsnummer: **0 153 593**
**B1**

(12) # EUROPÄISCHE PATENTSCHRIFT

(45) Veröffentlichungstag der Patentschrift: **19.11.87**

(51) Int. Cl.⁴: **C 07 D 251/32**

(21) Anmeldenummer: **85100842.5**

(22) Anmeldetag: **28.01.85**

(54) **Verfahren zur Herstellung von Cyanursäure.**

(30) Priorität: **11.02.84 DE 3404873**

(43) Veröffentlichungstag der Anmeldung: **04.09.85 Patentblatt 85/36**

(45) Bekanntmachung des Hinweises auf die Patenterteilung: **19.11.87 Patentblatt 87/47**

(84) Benannte Vertragsstaaten: **BE CH DE FR GB IT LI NL**

(56) Entgegenhaltungen:
**DE - A - 2 756 579**
**FR - A - 1 229 102**
**FR - A - 2 523 968**
**US - A - 2 943 088**
**US - A - 4 474 957**

(73) Patentinhaber: **HOECHST AKTIENGESELLSCHAFT, Postfach 80 03 20, D-6230 Frankfurt am Main 80 (DE)**

(72) Erfinder: **Pieper, Werner, Dr., Am Käferbruch 14, D-5014 Kerpen (DE)**

**EP 0 153 593 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Vorliegende Erfindung betrifft ein Verfahren zur Herstellung von granulierter Cyanursäure, indem man Harnstoff mit bereits gewonnener Cyanursäure in Gegenwart von Wasser erhitzt.

Cyanursäure, $(HNCO)_3$, wird technisch durch thermische Kondensation von Harnstoff gemäss folgender Reaktionsgleichung hergestellt:

$$3\,H_2N-\overset{\overset{\displaystyle O}{\|}}{C}-NH_2 \rightarrow (NHCO)_3 + 3\,NH_3$$

Die Kondensation kann unter Zuhilfenahme eines inerten Lösemittels (Nassverfahren) oder in Substanz (Trockenverfahren) durchgeführt werden [Ullmanns Ebcyklopädie der technischen Chemie, 4. Auflage, Band 9, S. 647 (1975)].

Nachteilig bei den Nassverfahren ist, dass stets eine Abtrennung der gebildeten Rohcyanursäure vom Lösemittel erforderlich ist, was mit zusätzlichem Arbeitsaufwand und zusätzlichen Kosten durch Lösemittelverluste usw. verbunden ist.

Bei den Trockenverfahren besteht dieses Problem nicht, doch muss hier ein Weg gefunden werden, das Zusammenbacken des Reaktionsgutes während der Umsetzung zu vermeiden, weil sich bei der Reaktion der Aggregatzustand des Kondensationsprodukts mit fortschreitender Reaktionszeit von flüssig über hochviskos zu fest ändert. Da der entstehende, schlecht wärmeleitende Feststoff die Heizflächen belegt, kommt die Kondensation bei geringen Cyanursäuregehalten des Produktes zum Stillstand. Als Ausweg wird die Umsetzung von Harnstoff oder anderen geeigneten Vorprodukten wie Biuret oder Harnstoffcyanurat in dünnen Schichten z.B. auf beheizten Bändern bzw. Drehtrommeln (US-A 3 030 641) oder auf Metallschmelzen (DE-C 1 065 420) beschrieben. Eine andere Möglichkeit ist, inerte, feste Verdünnungsmittel wie Cyanursäure zu benutzen und die Reaktion im Drehofen (US-A 2 943 088) oder in der Wirbelschicht (US-A 3 394 136) durchzuführen. Rückgutcyanursäure bietet sich dabei vorteilhaft an, da auf diese Weise kein systemfremder Stoff, der nachträglich wieder abgetrennt werden müsste, eingesetzt wird.

Gemäss dem Verfahren nach US-A 2 943 008 wird Harnstoff bei 240-360 °C unter ständigem Umwälzen mit einer bestimmten Geschwindigkeit zu einem freifliessenden Rohcyanursäure-Granulat kondensiert. Vor der eigentlichen Zersetzungsreaktion wird dabei der Harnstoff zu heisser Rückgutcyanursäure dosiert, so dass ein mit Harnstoff beschichtetes Einsatzprodukt entsteht, das 5-35% Harnstoff enthält. Der Harnstoff kann dabei in fester oder geschmolzener Form oder als heisse wässrige Lösung zu dem heissen Rohcyanursäuregranulat gemischt bzw. aufgesprüht werden. Im Falle des Aufsprühens einer heissen wässrigen Lösung wird vor dem Einsatz dieses Produkts in die eigentliche Thermolysereaktion das eingebrachte Wasser verdampft.

Die Rohcyanursäure aus diesen Prozessen enthält neben Cyanursäurevorstufen, wie Biuret und Triuret, unterschiedliche Mengen an Cyanursäureamiden, wie Ammelid, Ammelin und Melamin, welche die Cyanursäureausbeute herabsetzen und durch eine anschliessende Säuredigerierstufe entfernt bzw. zur Cyanursäure hydrolysiert werden müssen (FR-A-1 229 102).

Um diese Säuredigerierstufe zu umgehen und dennoch eine Rohcyanursäurequalität zu erzielen, die es erlaubt, das Rohprodukt für die Chlorierung zu Chlorisocyanuraten einzusetzen, wurden bislang Katalysatoren wie Ammoniumnitrat oder Salpetersäure der Eduktmischung zugesetzt (DE-A 2 756 579).

In der genannten DE-A 2 756 579 wird zwar darauf hingewiesen, dass 5% Wasser im Reaktionsgemisch, bezogen auf das Beschickungsgewicht des Harnstoffs, ohne nachteilige Ergebnisse toleriert werden können, doch war bislang nicht erkannt worden, dass, in Verbindung mit anderen, definierten Verfahrensmassnahmen, durch eine bestimmte Wassermenge im Ausgangsprodukt die Herstellung von Cyanursäure entscheidend vereinfacht und verbessert werden kann.

Überraschenderweise wurde nämlich gefunden, dass man den Einsatz des Harnstoffs in Form einer Schmelze sowie die Verwendung von Katalysatoren vermeiden kann, wenn man vorab aus granulierter Cyanursäure mit der im Endprodukt gewünschten Korngrösse Harnstoff und Wasser im Gewichtsverhältnis von 1,5 bis 3:1:0,04 bis 0,08, vorzugsweise von 2:1:0,05, eine Mischung herstellt und diese Mischung 10 bis 20 Stunden stehen lässt, bevor man sie in einem beheizten Rohr 5 bis 60 min lang unter ständigem Bewegen mit einer üblichen Dosiereinrichtung dem Heizsystem zuführt und auf Temperaturen von 270 bis 340 °C bis zur erfolgten Kondensation des Harnstoffs zu Cyanursäure erhitzt. Dieses Erhitzen kann in einem Röhrenofen und unter Normaldruck erfolgen. Als Cyanursäure setzt man vorzugsweise ein Produkt mit einer Korngrösse von 0,2-2 mm ein.

Als Röhrenofen kann z.B. ein Drehrohrofen oder ein feststehendes, beheiztes Rohr, durch welches das Reaktionsgut mit Hilfe einer Schnecke oder Spirale hindurchbefördert wird, benutzt werden. Als Cyanursäure kann bevorzugt Rückgutcyanursäure eingesetzt werden, die erfindungsgemäss im wesentlichen bereits als Granulat mit einem für die Umsetzung geeigneten Korndurchmesser anfällt. Die Mischung von Cyanursäure, Harnstoff und Wasser wird zweckmässigerweise mit einer üblichen Dosiereinrichtung, wie z.B. einer Dosierschnecke, kontinuierlich dem Heizsystem zugeführt, das auch einen Temperaturgradienten längs der Heizstrecke aufweisen kann. So kann z.B. die Temperatur am Anfang auf 270 °C, die am Ende auf 340 °C eingestellt werden. Niedrigere Temperaturen bewirken einen geringeren Umsetzungsgrad oder müssen durch längere Verweilzeiten kompensiert werden. Die Reaktionsdauer beträgt ca. 5 bis 60 min. Das Verhältnis Cyanursäure zu Harnstoff sollte aus ökonomischen Gründen möglichst klein gewählt werden, doch erhöht sich bei zu geringem Mischungsverhältnis die Gefahr der Verkrustung des Reaktionsrohres.

Führt man die Umsetzung ohne Zusatz von Wasser durch, agglomeriert ein grosser Teil der Offenbeschickung; es kommt zu Verkrustungen im Heizsystem, so dass es ohne Aufwendung zusätzlicher mechanischer Arbeit bald verstopft. Nur 30-40% des eingesetzten Produkts fallen dann in der gewünschten Granulatgrösse an. Ein Wasserzusatz von ca. 5 Gew.-%, bezogen auf eingesetzten Harnstoff, bewirkt dagegen, dass der Granulatanteil im Endprodukt auf 80 bis 90% ansteigt und die Innenwände des Heizrohrs bleiben dabei weitgehend frei von Anbackungen. Ein hoher Anteil an feinem Granulat im Endprodukt ist deshalb wünschenswert, da beispielsweise bei einer anschliessenden Weiterverarbeitung dessen Umsetzungsgrad höher ist als der von groben Agglomeraten. Wasserzusätze unter 3% Harnstoffgewicht zeigen einen zu geringen Effekt, wohingegen Wasserzusätze über 8 Gew.-% keinen weiteren Vorteil erbringen.

Die Strömung des bei der Kondensation gebildeten Ammoniak-Wasserdampf-Gemisches wird zweckmässigerweise durch einen geringen Inertgasstrom unterstützt, wodurch auch der kühlere Eingang des Erhitzungssystems kondensatfrei gehalten werden kann.

Die Vorteile des neuen Verfahrens seien anhand der folgenden Beispiele verdeutlicht:

*Beispiel 1*

Ein 120-cm-langes VA-Rohr mit einem Durchmesser von 2 cm, wird durch zwei elektrische Öfen beheizt. Ofen I hält 40 cm des Rohranfangs auf 270°C, während Ofen II die folgenden 60 cm auf 340°C erhitzt, so dass eine Heizzone von 100 cm resultiert. Eine Spirale mit einer Steilheit von 2 cm pro Windung liegt frei drehbar im Reaktionsrohr und wird über einen Motor mit 4 Umdrehungen pro Minute betrieben. Über eine Kolbendosierpumpe werden 4,8 g $min^{-1}$ des Eduktgemisches eindosiert, welches aus 578 g granulierter Cyanursäure (Korngrösse 0,2 bis 2 mm) und 298 g Harnstoff besteht. Die Verweilzeit des Eduktgemisches im Reaktionssystem beträgt theoretisch 12,5 min, tatsächlich werden jedoch bedingt durch Rückvermischung 20-30 min beobachtet. Es werden 671 g Produkt isoliert; nur 36% davon weisen eine Korngrösse von 0,2 bis 3 mm auf, während der grösste Teil zu groben Agglomeraten zusammengeklebt ist. Der Feinkornanteil zeigt einen Cyanursäuregehalt von 96,4%, der Grobkornanteil von 94,9% neben 1,6% Ammelid/Ammelin.

97 g des Produkts verbleiben als Verkrustung im Reaktionsrohr.

*Beispiel 2*

582 g Cyanursäure wie im Beispiel 1, 291 g Harnstoff und 14,6 g Wasser werden direkt nach dem Mischen mit 3 g $min^{-1}$ in das Reaktionsrohr eindosiert. Die Umsetzungsbedingungen und Verweilzeiten entsprechen den im Beispiel 1 genannten. 718 g Endprodukt werden isoliert; 77 Gew.-% davon weisen eine Korngrösse kleiner 2 mm auf mit einem Cyanursäuregehalt von 96 Gew.-% neben 1,5 Gew.-% Ammelid. 49 g des Produkts bleiben im Reaktionsrohr haften.

*Beispiel 3*

Analog Beispiel 2 werden 382 g Cyanursäure (0,2 bis 2 mm Korngrösse), 191 g Harnstoff und 9,6 g Wasser, jedoch erst 20 h nach dem Mischen, mit 4,7 g $min^{-1}$ in das Reaktionsrohr eindosiert; ansonsten entsprechen die Umsatzbedingungen und Verweilzeiten denen des Beispiels 1. Die Drehzahl der Transportschnecke im Rohr beträgt 4 Umdrehungen pro Minute. 475 g Endprodukt werden isoliert; 95 Gew.-% davon zeigen eine Korngrösse von 0,2 bis 2 mm. Der Cyanursäuregehalt wird mit 96 Gew.-%, der Ammelidgehalt mit 2 Gew.-% bestimmt.

19 g des Produkts verbleiben als Verkrustung im Reaktionsrohr.

*Beispiel 4*

Analog Beispiel 2 werden 285 g Cyanursäure, 142 g Harnstoff und 7 g Wasser, jedoch erst 15 h nach dem Mischen, mit 3,1 g pro Minute in das Raktionsrohr dosiert. Die Drehzahl der Transportschnecke beträgt 20 Umdrehungen pro Minute. Die Verweilzeit des Produkts im Rohr beträgt ca. 5 min.

334 g Endprodukt werden isoliert neben 24 g Verkrustung im Rohr. Der Cyanursäuregehalt beträgt 98%, der Ammelidgehalt 0,8%.

**Patentansprüche**

1. Verfahren zur Herstellung von granulierter Cyanursäure, indem man Harnstoff mit bereits gewonnener Cyanursäure in Gegenwart von Wasser erhitzt, dadurch gekennzeichnet, dass man

a) granulierte Cyanursäure mit der im Endprodukt gewünschten Korngrösse einsetzt;

b) aus dieser Cyanursäure, Harnstoff und Wasser im Gewichtsverhältnis von 1,5 bis 3:1:0,04 bis 0,08 vorab eine Mischung herstellt und

c) diese Mischung 10 bis 20 Stunden stehen lässt, bevor man sie in einem beheizten Rohr 5 bis 60 min lang, unter ständigem Bewegen mit einer üblichen Dosiereinrichtung kontinuierlich dem Heizsystem zuführt und dabei auf Temperaturen von 270 bis 340°C bis zur erfolgten Kondensation des Harnstoffs zu Cyanursäure erhitzt.

2. Verfahren nach Anspruch 1, dadurch gekennzeichnet, dass man eine Mischung von Cyanursäure, Harnstoff und Wasser im Gewichtsverhältnis von 2:1:0,05 einsetzt.

**Claims**

1. Process for making granulated cyanuric acid by heating urea with previously produced cyanuric acid, in the presence of water, which comprises:

a) using granulated cyanuric acid having the particle size targeted for the final product;

b) initially preparing a mixture from the said cyanuric acid, urea and water in a ratio by weight of 1.5-3:1:0.04-0.08; and

c) allowing the said mixture to stand over a period of 10 to 20 hours prior to delivering it, inside a heated tube, with the aid of a customary dosing means with continuous agitation and within 5-60 min to the heating system, and heating it to 270-340°C until the urea commences condensing to cyanuric acid.

2. Process as claimed in claim 1, wherein a

mixture of cyanuric acid, urea and water in a ratio by weight of 2:1:0.05 is used.

**Revendications**

1. Procédé pour la fabrication d'acide cyanurique granulé par chauffage d'urée avec de l'acide cyanurique déjà produit en présence d'eau, caractérisé en ce que:

a) on utilise de l'acide cyanurique granulé présentant la granulométrie recherchée pour le produit final;

b) on prépare initialement un mélange de cet acide cyanurique, d'urée et d'eau dans les proportions pondérales 1,5 à 3:1:0,04 à 0,08 et

c) on laisse reposer ce mélange pendant 10 à 20 h avant de l'amener en continu à l'aide d'un dispositif doseur habituel, sous agitation continue et en 5 à 60 min dans un tube chauffé au système chauffant en le portant à des températures de 270 à 340°C jusqu'à condensation de l'urée en acide cyanurique.

2. Procédé selon la revendication 1, caractérisé en ce que l'on utilise un mélange d'acide cyanurique, d'urée et d'eau dans les proportions pondérales 2:1:0,05.